# EUROPEAN PATENT SPECIFICATION

(11) **EP 2 505 680 B1**
(45) Date of publication and mention of the grant of the patent: **06.05.2015**
(21) Application number: 11160537.4
(22) Date of filing: 30.03.2011
(51) Int. Cl.: C22C 9/00, A47C 31/00, A43B 13/10, A43B 17/04, D06M 11/83

(54) **Copper metal alloy**
Kupfermetallegierung
Alliage métallique de cuivre

(43) Date of publication of application: 03.10.2012
(73) Proprietor: MEDIKA S.r.l., 36057 Arcugnano (VI) (IT)
(72) Inventor: Amenduni Gresele, Massimo, 36100 Vicenza (IT); Penna, Rodolfo, 31100 Treviso (IT)
(74) Representative: Notaro, Giancarlo

(56) References cited:
- EP-A2- 0 166 144
- DE-A1- 19 629 376
- JP-A- 4 339 590
- JP-A- 56 102 395
- JP-A- 63 154 290
- US-A- 3 451 808
- US-A1- 2009 317 291

## Description

The present invention regards a metal element made of a copper metal alloy, useable in particular for making elements for promoting and/or stimulating vital energy in organisms, in particular in human beings, animals and plants.

The beneficial properties of some metals, and in particular copper, for the wellbeing of organisms have been known over the years. Devices of various types exploiting such properties are for example described in JP-A-2005/253934, WO-A-8400298, JP-A-2007/203000, WO-A-95/09591.

EP 0 166 144 discloses a homogeneous ductile copper-based alloy having a metastable structure used as a brazing filler in the form of a foil or a powder.

The object of the present invention is to provide a metal element made of a metal alloy having such properties in high amounts and which is suitable to be used in various applications, as disclosed in the claims.

In order to achieve such object, the invention mainly aims at providing an a metal element for promoting and/or stimulating vital energy in organisms, in particular in human beings, animals and plants, said element being made of a copper metal alloy consisting of:
- between 60% and 70% in weight of copper (Cu),
- between 16% and 24% in weight of silver (Ag),
- between 3% and 6% in weight of zinc (Zn),
- between 3% and 6% in weight of manganese (Mn),
- between 0.5% and 6% in weight of germanium (Ge).
- optionally between 3% and 6% in weight of tin (Sn),
- optionally between 0.1% and 5% in weight of platinum (Pt).

Studies and experiments conducted by the applicant revealed that the alloy according to the invention can be advantageously used for making elements and devices beneficial to the wellbeing of human beings, animals or plants, from different points of view.

The present invention also particularly aims at providing a metal sheet, for example in form of a plate or extended strip, as an element for promoting and/or stimulating energy, characterised in that it is constituted by a copper metal alloy of the previously defined type. Studies and tests conducted by the applicant revealed that a plate-like configuration of the sheet, rectangular-shaped and having rounded vertices, thickness comprised between 0.10 mm and 1 mm, preferably equivalent to 0.50 mm, is particularly advantageous. Still in the preferred embodiment of the abovementioned plate-shaped sheet, the plate has sides each having length comprised between 8 mm and 30 mm, preferably measuring 8 x 8 mm.

A particularly advantageous application of the sheet according to the invention is constituted by an inner sole for footwear, on which one or more of said plates are arranged.

Studies and experiments have revealed that the beneficial effects of the application of the invention on an inner sole for footwear are particularly enhanced in cases where said plates are positioned at predefined points of the plantar of the foot, which are particularly important for the wellbeing of the person according to the studies available in the field of reflexology.

A further advantageous application of a sheet constituted by the metal alloy according to the invention is that in which one or more sheets are included in piece of clothing, for example stockings or undershirts, so as to be adjacent to the body of the person wearing such clothes.

Still, another advantageous application is that in which one or more sheets constituted by the metal alloy according to the invention are included in mattresses or padding elements for beds, seats or armchairs. In this case, the sheets are preferably in form of extended strips, with width comprised between 8 mm and 30 mm and length up to 2000 mm.

Further advantageous applications are constituted orthopaedic collars, plasters, orthopaedic elastic bandages.

Elements constituted by the metal alloy according to the invention can even be applied to articles for playing or pastime, for example articles of the type comprising a pendulum element which is oscillated to obtain indications of various types or predictions.

Further characteristics and advantages of the invention will be apparent from the description that follows with reference to the attached drawings, provided purely by way of non-limiting example, wherein:
figure 1 illustrates a perspective view of an embodiment of a sheet constituted by the metal alloy according to the invention, and
figure 2 is a plan view of an inner sole for footwear incorporating a plurality of plate-like sheets of the type illustrated in figure 1.

As illustrated above, the applicant, through subsequent studies and experiments, identified a new composition for a copper metal alloy which allows obtaining optimal results to provide elements intended to be used in association to organisms, in particular human beings, to obtain several beneficial effects.

Also as indicated above, the invention provides for a first embodiment in which the metal alloy consists of the components indicated in table 1. Such table indicates the minimum and maximum conditions of the range of possible variation of the percentage in weight of each element.

**Table 1**

| Element | % min | % max |
|---|---|---|
| Cu | 60 | 70 |
| Ag | 16 | 24 |
| Zn | 3 | 6 |
| Mn | 3 | 6 |
| Sn | 3 | 6 |
| Ge | 0.5 | 6 |

A second embodiment of the metal alloy according to the invention is indicated in the following table 2, which is characterised - with respect to table 1 - by the absence of tin.

**Table 2**

| Element | % min | % max |
|---|---|---|
| Cu | 60 | 70 |
| Ag | 16 | 24 |
| Zn | 3 | 6 |
| Mn | 3 | 6 |
| Ge | 0.5 | 6 |

A third embodiment of the metal alloy according to the invention is also characterised by the absence of tin and it also contains platinum, as indicated in the following table 3.

**Table 3**

| Element | % min | % max |
|---|---|---|
| Cu | 60 | 70 |
| Ag | 16 | 24 |
| Zn | 3 | 6 |
| Mn | 3 | 6 |
| Ge | 0.5 | 6 |
| Pt | 0.1 | 5 |

An embodiment in which the alloy comprises both tin and platinum is also provided for, like in table 4

**Table 4**

| Element | % min | % max |
|---|---|---|
| Cu | 60 | 70 |
| Ag | 16 | 24 |
| Zn | 3 | 6 |
| Mn | 3 | 6 |
| Ge | 0.5 | 6 |
| Sn | 3 | 6 |
| Pt | 0.1 | 5 |

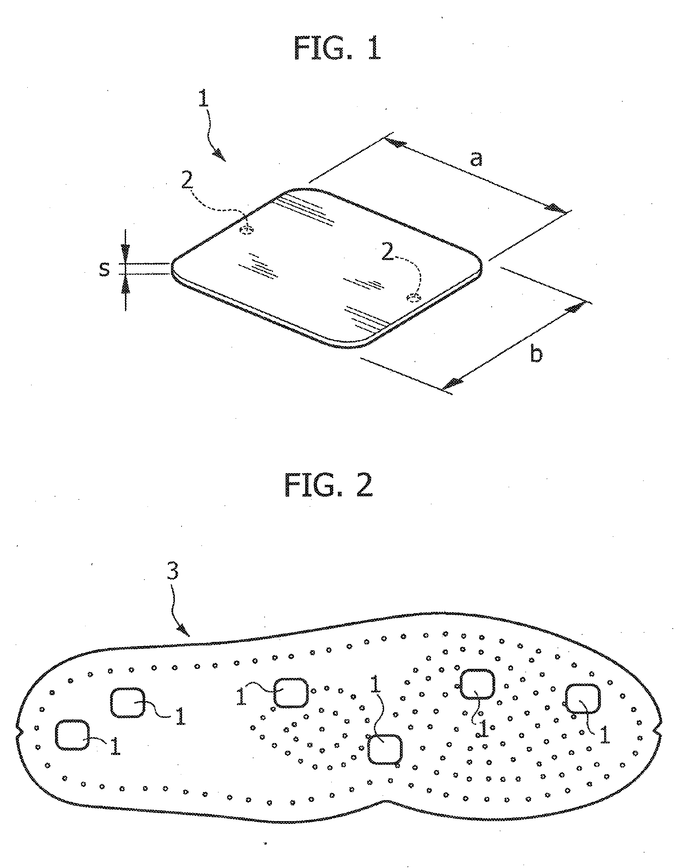

According to the invention, any of the abovementioned compositions is used to obtain - through casting according to any known technique - elements in form of metal sheets, for example in forma of plates of the type illustrated in figure 1 of the attached drawings. In such figure, the plate constituted by the metal alloy according to the invention, substantially rectangular flat-shaped, having sides a, b and thickness s, is indicated in its entirety with reference number 1. The vertices of the plate are preferably rounded. A thickness s comprised between 0.10 mm and 1 mm, preferably equivalent to 0.50 mm, is advantageous. The sides a,b may each have for example a length comprised between 8 mm and 30 mm. A typical dimension may be 8 x 8 mm. Should the plate 1 be intended to be fixed by stitching to a clothing item or a fabric substrate, the plate should be provided with at least one pair of holes 2 (indicated with a dashed line in figure 1) for engaging a stitching thread.

Figure 2 shows an application example of the metal element according to the invention. In such figure, an inner sole for footwear which is provided with a plurality of plates 1 of the type illustrated in figure 1 is indicated in its entirety with reference number 3. The plates 1 are fastened to the inner sole 3 through any known technique, for example by means of an adhesive. Arranging the plates 1 adjacent to the plantar of the foot of the person wearing the footwear leads to, as revealed by tests conducted by the applicant, various beneficial effects. Such effects are also observed by arranging plates of the type of figure 1 obtained using the metal alloy according to the invention adjacent to other parts of the body of the person, for example incorporating one or more plates in plasters, or in piece of clothing of various types (for example in stockings and undershirts), or even in orthopaedic elastic bandages. A series of specific tests were conducted with reference to an orthopaedic collar including a plurality of plates of the type of figure 1.

A further advantageous application is that in which a plurality of sheets constituted by the metal alloy of the invention and preferably obtained in form of extended strips, are incorporated in mattresses or padding elements for beds, seats or armchairs so as to be adjacent to the back or other parts of the body of the person. In this case, the strip has a width for example comprised between 8 mm and 30 mm and length up to 2000 mm.

The various observed advantageous effects in particular include:
- marked increase of the neuromuscular performance with an ensuing lower tendency to tire from walking, with a marked increase of the threshold of tiredness;
- antalgic action in particular against pains related to the osteoarticular and musculoskeletal system, including myofascial syndromes and night muscle cramps;
- antalgic actions against pains related to sciatica;
- improvement of cutaneous microcirculation, and presumably also muscular, in the lower limbs, beneficial for example in case of diabetic foot;
- in some emblematic cases of serious skin diseases of eczematous-psoriasic type, with disappearance of the abovementioned skin lesions upon using the device;
- greater stability and motion equilibrium secondary to a presumable postural optimisation, even in case of severe Parkinson's disease.

Furthermore, the tests revealed that the beneficial action becomes more efficient directly proportionally to the seriousness of the base disease and the correlated symptoms. In other words the positive effect of the metal element made of the alloy according to the invention is more noticeable in cases where it is required more.

Tests incorporating plates constituted by the metal alloy according to the invention in stockings worn by people going for long journeys by air were also carried out, observing a substantial reduction of the swelling of the lower limbs deriving from long periods in pressurized cabins. The use of sheets constituted by the metal alloy according to the invention in mattresses or padding elements for beds, armchairs or the like, revealed a substantial relaxing effect for the person, with beneficial effects even in case of insomnia.

As illustrated above, elements constituted by the metal alloy according to the invention can also be applied to various types of articles useable for playing or pastime. For example, a concrete application of this type is that regarding a device with a pendulum element useable for prediction.

## Claims

1. A metal element for promoting and/or stimulating vital energy in living organisms, in particular in human beings, animals and plants, said element being made of a copper metal alloy, **characterised in that** said alloy consists of :
- between 60% and 70% in weight of copper (Cu),
- between 16% and 24 % in weight of silver (Ag),
- between 3% and 6% in weight of zinc (Zn),
- between 3% and 6% in weight of manganese (Mn),
- between 0.5% and 6% in weight of germanium (Ge),
- optionally between 3% and 6% in weight of tin (Sn),
- optionally between 0.1% and 5% in weight of platinum (Pt).

2. A metal element according to claim 1, **characterised in that** it is a sheet in form of a plate or strip..

3. A metal element according to claim 2, **characterised in that** it has a thickness comprised between 0.1 mm and 1 mm, preferably equivalent to 0.50 mm.

4. A metal element according to claim 2, **characterised in that** it is rectangular-shaped with rounded vertices and sides each having length comprised between 8 mm and 30 mm, preferably measuring 8 x 8 mm.

5. A metal element according to claim 4, **characterised in that** it has one or more holes for engaging a stitching thread.

6. A metal element according to claim 3, **characterised in that** it is in form of a strip, with width comprised between 8 mm and 30 mm and length up to 2000 mm.

7. Inner sole for footwear, **characterised in that** it includes one or more metal elements according to one of claims 2-5.

8. Piece of clothing, **characterized in that** it includes one or more metal elements according to one of claims 2-5.

9. Mattress or padded element for a bed, seat or armchair, **characterized in that** it includes one or more metal elements according to one of claims 2-6.

10. Orthopaedic collar, **characterized in that** it includes one or more metal elements according to one of claims 2-5.

11. Plaster, **characterised in that** it includes one or more metal elements according to one of claims 2-5.

12. Orthopaedic elastic bandage, **characterised in that** includes one or more metal elements according to one of claims 2-5.

## Patentansprüche

1. Metallelement zum Fördern und/oder Anregen der Lebensenergie in lebenden Organismen, insbesondere im Menschen, in Tieren und Pflanzen, wobei das Element aus einer Kupfermetalllegierung besteht,
**dadurch gekennzeichnet, dass** die Legierung aus
- zwischen 60 und 70 Gewichtsprozent Kupfer (Cu),
- zwischen 16 und 24 Gewichtsprozent Silber (Ag),
- zwischen 3 und 6 Gewichtsprozent Zink (Zn),
- zwischen 3 und 6 Gewichtsprozent Mangan (Mn),
- zwischen 0,5 und 6 Gewichtsprozent Germanium (Ge),
- wahlweise zwischen 3 und 6 Gewichtsprozent Zinn (Sn),
- wahlweise zwischen 0,1 und 5 Gewichtsprozent Platin (Pt).
besteht.

2. Metallelement nach Anspruch 1, **dadurch gekennzeichnet, dass** es sich um ein Blech in Form einer Platte oder eines Streifens handelt.

3. Metallelement nach Anspruch 2, **dadurch gekennzeichnet, dass** es eine Dicke von zwischen 0,1 mm und 1 mm, vorzugsweise gleich 0,50 mm aufweist.

4. Metallelement nach Anspruch 2, **dadurch gekennzeichnet, dass** es die Form eines Rechtecks aufweist, mit abgerundeten Eckpunkten und Seiten, die jeweils eine Länge von zwischen 8 mm und 30 mm aufweisen, vorzugsweise 8 x 8 mm messen.

5. Metallelement nach Anspruch 4, **dadurch gekennzeichnet, dass** es ein oder mehrere Löcher aufweist, um in Eingriff mit einem Heftgarn zu gelangen.

6. Metallelement nach Anspruch 3, **dadurch gekennzeichnet, dass** es die Form eines Streifens aufweist, mit einer Breite von zwischen 8 mm und 30 mm und einer Länge von bis zu 2000 mm.

7. Innensohle für Schuhwerk, **dadurch gekennzeichnet, dass** sie eines oder mehrere Metallelemente gemäß einem der Ansprüche 2 bis 5 umfasst.

8. Kleidungsstück, **dadurch gekennzeichnet, dass** es eines oder mehrere Metallelemente gemäß einem der Ansprüche 2 bis 5 umfasst.

9. Matratze oder Polsterelement für ein Bett, einen Sitzplatz oder Sessel, **dadurch gekennzeichnet, dass** es eines oder mehrere Metallelemente gemäß einem der Ansprüche 2 bis 6 umfasst.

10. Orthopädischer Kragen, **dadurch gekennzeichnet, dass** er eines oder mehrere Metallelemente gemäß einem der Ansprüche 2 bis 5 umfasst.

11. Pflaster, **dadurch gekennzeichnet, dass** es eines oder mehrere Metallelemente gemäß einem der Ansprüche 2 bis 5 umfasst.

12. Orthopädische Elastikbandage, **dadurch gekennzeichnet, dass** sie eines oder mehrere Metallelemente gemäß einem der Ansprüche 2 bis 5 umfasst.

## Revendications

1. Élément métallique pour favoriser et/ou stimuler l'énergie vitale dans les organismes vivants, en particulier chez les êtres humains, les animaux et les plantes, ledit élément étant réalisé en un alliage métallique de cuivre, **caractérisé en ce que** ledit alliage est constitué :
- entre 60% et 70% en poids de cuivre (Cu),
- entre 16% et 24% en poids d'argent (Ag),
- entre 3% et 6% en poids de zinc (Zn),
- entre 3% et 6% en poids de manganèse (Mn),
- entre 0,5% et 6% en poids de germanium (Ge),
- facultativement entre 3% et 6% en poids d'étain (Sn),
- facultativement entre 0,1% et 5% en poids de platine (Pt).

2. Élément métallique selon la revendication 1, **caractérisé en ce qu'**il représente une feuille en forme de plaque ou de bande.

3. Élément métallique selon la revendication 2, **caractérisé en ce qu'**il présente une épaisseur comprise entre 0,1 mm et 1 mm, de préférence équivalente à 0,50 mm.

4. Élément métallique selon la revendication 2, **caractérisé en ce qu'**il présente une forme rectangulaire avec des sommets arrondis et des côtés ayant chacun une longueur comprise entre 8 mm et 30 mm, de préférence mesurant 8 x 8 mm.

5. Élément métallique selon la revendication 4, **caractérisé en ce qu'**il comporte un ou plusieurs trou(s) pour engager un fil de couture.

6. Élément métallique selon la revendication 3, **caractérisé en ce qu'**il a la forme d'une bande, avec une largeur comprise entre 8 mm et 30 mm et une longueur allant jusqu'à 2000 mm.

7. Semelle intérieure pour chaussure, **caractérisée en ce qu'**elle comporte un ou plusieurs élément(s) métallique(s) selon l'une des revendications 2 à 5.

8. Vêtement, **caractérisé en ce qu'**il comporte un ou plusieurs élément(s) métallique(s) selon l'une des revendications 2 à 5.

9. Matelas ou élément rembourré pour un lit, un siège ou un fauteuil, **caractérisé en ce qu'**il comporte un ou plusieurs élément(s) métallique(s) selon l'une des revendications 2 à 6.

10. Collier orthopédique, **caractérisé en ce qu'**il comporte un ou plusieurs élément(s) métallique(s) selon l'une des revendications 2 à 5.

11. Plâtre, **caractérisé en ce qu'**il comporte un ou plusieurs élément(s) métallique(s) selon l'une des revendications 2 à 5.

12. Bandage élastique orthopédique, **caractérisé en ce qu'**il comporte un ou plusieurs élément(s) métallique(s) selon l'une des revendications 2 à 5.
